# EUROPEAN PATENT APPLICATION

(11) **EP 0 934 944 A1**
(43) Date of publication of application: **11.08.1999**
(21) Application number: 98124764.6
(22) Date of filing: 28.12.1998
(51) Int. Cl.: C07D 501/00, A61K 31/545

(54) **7-bromo- and 7,7-dibromo-cepham and cephem derivatives and their use as protease inhibitors**

(30) Priority: 30.12.1997 HR 970713
(71) Applicant: PLIVA farmaceutska, kemijska, prehrambena i kozmeticka industrija, dionicko drustvo, 10000 Zagreb (HR)
(72) Inventor: Lukic, Irena, 10000 Zagreb (HR)
(74) Representative: von Füner, Alexander, Prof.h.c. Dr.

(57) **Abstract**

The object of the present invention relates to novel 7-bromo- and 7,7-dibromo-cepham and 7-bromo- and 7,7-dibromo-cepham derivates of general formula I 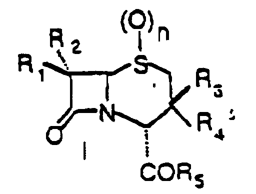 wherein the radicals have the meanings
- R₁: is hydrogen or bromine,
- R₂: is hydrogen or bromine,
- R₃: is bromine,
- R₄: is methyl, hydroxy,
- R₅: is -OR₆ or -NR₇R₈, wherein R₆, R₇ and R₈ are hydrogen, alkyl, alkylaryl,
- n =: 0, 1, 2,
and of general formula II 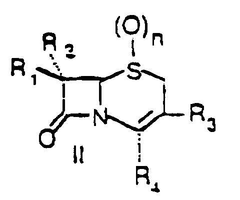 wherein the radicals have the meanings
- R₁: is hydrogen or bromine,
- R₂: is hydrogen or bromine,
- R₃: is methyl,
- R₄: is hydrogen, -COOR₅ or CONR₆R₇, wherein R₅, R₆ and R₇ are hydrogen, alkyl, alkylaryl,
- n =: 0, 1, 2.

According to the present invention novel compounds are potentially active as inhibitors of proteases, especially humen leukocyte elastases (HLE).

## Description

- IPC:: C 07 D 501/00
A 61 K 31/545
C 07 D 501/62

The object of the present invention relates to novel 7-bromo- and 7,7-dibromo-cepham and cephem derivatives, to a process for the preparation thereof and to pharmaceutical and veterinary formulations containing them. According to the present invention novel compounds are potentially useful as inhibitors of proteases, especially human leukocyte elastases (HLE) and for the prophylaxis, control and treatment of diseases including uncontrolled release of proteolytic enzymes, especially of emphysema, metastasis, cystic fibrosis.

Methyl 7,7-dibromo-3-methyl ceph-3-em-4-carboxylate was obtained as the only product of rearrangement of methyl 6,6-dibromopenicillanate-1-beta sulfoxide in refluxing xylene under the addition of a small amount of p-toluenesulfonic acid. (R. B. Morin, B. G. Jackson, R. A. Mueller, E. R. Lavagnino, W. B. Sconlin, and S. L. Andrews; J. Am. Chem. Soc. 1969, 91, 1401).

Pummer's rearrangement in acetic anhydride was carried out on methyl 6,6-dibromopenicillanate-1-beta sulfoxide, whereby a mixture of 2-acetoxy-methylpenam, 3-acetoxycepham and methyl 7,7-dibromo-3-methyl ceph-3-em-4-carboxylate was obtained (J. Peter Clayton, John H. C. Nayler, Michael J. Pearson and Robert Soughte, Beacham Research Laboratories; J. Chem. Soc. Perkin. Trans I 1974, 22-25).

By an electrolysis of Kamiya's disulfide i.e. 3-phenylacetamido-4-(2-benzothiazolyldithi)-azetidinone with sodium iodide there 7-phenylacetamido-3-beta-iodo-3-alpha-methyl cepham was obtained, which was dehydrohalogenated into 3-methyl cephem (Torii, Sigeru; Tanaka, Hideo; Siroi, Takashi; Sasaoka, Michio; Saitoh, Norio; Nokami, Junzo; Tada, Nobuhito: Chem. Lett. 1982 (11) 1829-32).

By a reaction of metal fluorides with asymmetric azetidinone disulfide in methylene chloride and by subsequent oxidation, 3-beta fluoro-3-alpha-methyl-cephams and 1-oxides were obtained, the latter, however, with phenoxyacetamide group in position 7. Yet by a reaction of 2-beta-halomethylpenams with silver fluoride, 3-beta fluoro-3-alpha-methyl 7-phenoxyacetamido cephams were obtained (Micetich, Roland G.; Betageri, Sucheta; Singh, Rajeshwar; Payne, Pamela: J. Org. Chem. 1987, 52(5) 915-18).

Esters of 3-bromo-3-methyl 7-phenylacetamido and 7-phenoxyacetamido cepham were prepared by the group of Fujisawa (Kamiya T., Teraji T., Saito Y., Hashimoto M., Nakaguchi O. and Oku T.: Tetrahedron Lett. 1973, 3001; GB 1,453,301), and exocyclic dehydrobromation thereof was used for the preparation of cefaclor.

The expansion of 6-phenoxyacetamido-2-beta-bromomethyl-penam into 3-methyl-cepham was carried out with triphenyltin hydride (Baldwin, Jack E.; Adlington Robert M.; Kang, Tae Won; Lee, Eun; Scofield, Christopher J.: Tetrahedron 1988, 44 (18) 5953-7). The same product was prepared by homolytical debromation of 3-beta-bromo-3-alpha-methyl 7-phenoxyacetamido cepham with triphenyltin hydride (Baldwin, Jack E.; Adlington, Robert M.; Kang, Tae Won; Lee, Eun; Scofield, Christopher J.: J. Chem. Soc. Chem. Commun. 1987, (2) 104-6).

3-Methyl-3-halo-7-phenoxyacetamido cepham sulfoxides in reactions with metal salts, especially mercuric salts, give 3-methylen-cepham compounds. (Corfield J. R. and Taylor, C. G., 1978, Tetrahedron Lett. 2915).

In the literature compounds of similar structures are described as inhibitors of elastases (Bissolino, P.; Alpegiani, M.; Corigli, R.; Perrone, E.; Pharmacia & Upjohn S.p.A.; WO 97/02268, 23.01.1997, PCT/EP96/02883; Grigory Veinberg et al.: Bioorganic & Medicinal Chemistry Letters. Vol.7, 843-6, 1997; Alpegiani M. et al.: Bioorganic & Medicinal Chemistry Letters Vol. 2, 1127-1132, 1992; Paul E. Finke et al.: Journal of Medicinal Chemistry, 1992, Vol. 35, 3731-3744.)

According to our knowledge of prior art, 7-bromo- and 7,7-dibromo-cepham and cephem derivatives have not been kown.

The object of the present invention relates to 7-bromo- and 7,7-dibromo-cepham derivatives of general formula **I** 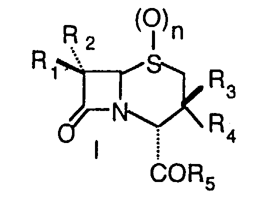 wherein
- R₁: is hydrogen or bromine,
- R₂: is hydrogen or bromine,
- R₃: is bromine,
- R₄: is methyl, hydroxy,
- R₅: is -OR₆ or -NR₇R₈, wherein R₆, R₇ and R₈ are hydrogen, alkyl, alkylaryl,
- n =: 0, 1, 2,
and to 7-bromo- and 7,7-dibromo-cephem derivatives of general formula **II** 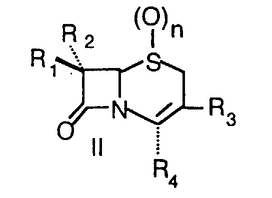 wherein
- R₁: is hydrogen or bromine,
- R₂: is hydrogen or bromine,
- R₃: is methyl,
- R₄: is hydrogen, -COOR₅ or CONR₆R₇, wherein R₅, R₆ and R₇ are hydrogen, alkyl, alkylaryl,
- n =: 0, 1, 2.

A further object of the present invention is a process for the preparation of 7-bromo- and 7,7-dibromo-cepham derivatives of the general formula **I**, and 7-bromo- and 7,7-dibromo-cephem derivatives of the general formula **II**, wherein the radicals have the above given meanings and which can be prepared from esters or amides of 6-bromo- and 6,6-dibromopenicillanic acid sulfoxides of general formula **III** 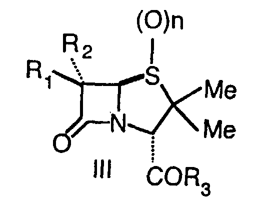 wherein
- R₁: is hydrogen or bromine,
- R₂: is hydrogen or bromine,
- R₃: is OR₄ or NR₅R₆, wherein R₄, R₅ and R₆ are hydrogen, alkyl, alkylaryl,
- n =: 1,
by heating at boiling temperature in some organic solvent e.g. acetonitrile, toluene or some other solvent, with or without an addition of tetrabutylammonium bromide and by subsequent evaporation of the reaction mixture to a dry residue, which is after treatment purified by flash chromatography or chomatography on a silica gel column or is crystallized from a suitable solvent or from a suitable mixture of solvents and then a product is isolated or the obtained product is immediately oxidized by usual agents e.g. by hydrogen peroxide or potassium permanganate, with or without an addition of glacial acetic acid or formic acid, whereat there are formed 7-bromo- and 7,7-dibromo-cepham derivatives of the general formula **I**, wherein
- R₁: is hydrogen or bromine,
- R₂: is hydrogen or bromine,
- R₃: is bromine,
- R₄: is methyl, hydroxy,
- R₅: is -OR₆ or -NR₇R₈, wherein R₆, R₇ and R₈ are hydrogen, alkyl, alkylaryl,
- n =: 0, 1, 2,
and 7-bromo- and 7,7-dibromo-cephem derivatives of the general formula **II**, wherein
- R₁: is hydrogen or bromine,
- R₂: is hydrogen or bromine,
- R₃: is methyl,
- R₄: is hydrogen, -COOR₅ or CONR₆R_{7,} wherein R₅, R₆ and R₇ are hydrogen, alkyl, alkylaryl,
- n =: 0, 1, 2.

Derivatives of 6,6-dibromopenicillanic acid were prepared from 6-amino-penicillanic acid according to known processes (R. A. Volkmann, R. D. Carroll, R. B. Drolet, M. L. Elliott and B. S. Moore., J. Org. Chem. 47, (1982) 3344-5; and Wayne E. Barth, US 4,234,579, 18 Nov 1980). Amides were prepared according to Mascaretti O. et al., J. Chem. Research ( 14) 1988, 1501-36.

Sulfoxides of amides were prepared in one phase by acid chloride or mixed anhydride methods with subsequent amidation and oxidation of the obtained product with m-chloroperbenzoic acid or with hydrogen peroxide under addition of acetic acid (R. Micetich, Heterocycles 22, 531, (1984)).

An object of the present invention are newly synthesised substances prepared from esters or amides of 6-bromo- and 6,6-dibromo-penicillanic acid sulfoxides. Another object of the present invention is a novel process, which is simple and easy to be carried out.

A further object of the present invention relates to the use of these compounds as active components in pharmaceutical preparations with antibacteric or synergistic action or as inhibitors of elastases.

The invention is illustrated by the following examples, which do not limit the scope of the invention in any way.

### Example 1

### 6,6-Dibromopenicillanic acid benzylamide

To a solution of 6,6-dibromopenicillanic acid (7.2 g; 0.02 mole) in methylene chloride (100 ml, dry), cooled with ice, at 0°C, triethylamine (2.8 ml; 0.02 mole) was added and it was stirred at 0°C for half an hour. Then ethyl chloroformiate (2.1 ml; 0.02 mole) was added and it was stirred in ice for further half an hour. Then a solution of benzylamine (4.8 ml; 0.044 mole; in methylene chloride (50 ml)) was added dropwise to pH 7.5 (used 3.74 ml; 0.034 mole) and stirred at room temperature for further two hours. The precipitate was sucked off and the liquor was rinsed with 1N HCl, with water, dried and evaporated to dryness (9.8 g). The obtained residue was purified by flash chromatography with a solvent system petroleum ether-methylene chloride (7:3), then petroleum ether-methylene chloride (1:1) and then with methylene chloride, whereby 1.98 g (22.1%) of a product were isolated.
M.p. 156-158°C
Rf 0.3 (methylene chloride)
IR (KBr) νₘₐₓ (CDCl₃): 1810 (β-lactam) and 1660 (amide) cm⁻¹.
¹H NMR (CDCl₃, 300 MHz): 1.48 (3H, s, 13 Me), 1.67 (3H, s, 14 Me), 4.34 (1H, s, 3-H), 4.35 and 4.57 (each 1H, ABX system, J _{gem.} 14.5 Hz, J _{vic} 5.7 and 6.0 Hz resp., 11H), 5.66 (1H, s, 5-H), 6.66 (1H, br t, NH), 7.31 (5H, s, Ph) ppm.
¹³C NMR (CDCl₃, 300 MHz) δ: 26.2 (C-10), 30.3 (C-9), 43.4 (C-11), 55.0 (C-6), 64.6 (C-2), 71.3 (C-3), 78.5 (C-5), 127.6 and 128.7 (C-2' to C-6'), 137.0 (C-1') 165.9 (C-7), and 166.4 (C-8) ppm.

| | | | |
|---|---|---|---|
| Found: | C 40.1; | H 3.5; | N 6.3 |
| C₁₅H₁₆Br₂N₂O₂S: | C 40.2; | H 3.6; | N 6.2 |

### Example 2

### Benzylamide of 6,6-dibromopenicillanic acid sulfoxide

6,6-Dibromopenicillanic acid benzylamide (4.48 g, 0.01 mole) was dissolved in methylene chloride (140 ml), then glacial acetic acid (22.8 ml; 0.400 mole) and hydrogen peroxide (12.5 ml, 0.120 mole) were added and it was stirred at room temperature for 20 hours. When the reaction was completed, it was rinsed with water, NaHCO₃, salty water, dried and evaporated to dryness. Obtained were 4.2 g of a product, which were treated with methanol, the precipitate (2.2 g) was sucked off and the evaporated liquor was purified by flash chromatography with methylene chloride and methylene chloride-acetone (10:0.3), whereby more white crystals (0.39 g) were obtained. Totally, there were obtained 2.59 g (55.75%) of the product.
M.p. 158-160 °C (MeOH)
Rf 0.20 in a system methylene chloride-acetone (10:0.3)
IR (KBr) νₘₐₓ: 3305 (s), 1810 (vs), 1670 (s), 1535 (s), 1275 (vs), 1065 (vs) cm⁻¹.
¹H NMR (CDCl₃, 300 MHz) δ: 1.41 (3H, s, Me), 1.69 (3H, s, Me), 4.42 (1H, s, 3-H), 4.38 and 4.61(each 1H, ABX system, J _{gem.} 14.8 Hz, J _{vic} 5.9 and 6.0 Hz resp., 11H), 5.04 (1H, s, 5-H) 6.75 (1H, br s, NH), 7.32 (5H, s, Ph) ppm.

| | | | |
|---|---|---|---|
| Found: | C 39.2; | H 3.5; | N 6.1 |
| C₁₅H₁₆Br₂N₂O₃S: | C 38.9; | H 3.5; | N 6.0 |

### Example 3

### Benzylamide of 6,6-dibromopenicillanic acid sulfoxide

To a solution of 6,6-dibromopenicillanic acid (14.4 g; 0.04 mole) in methylene chloride (200 ml, rinsed, dry), cooled with ice, at 0°C, triethylamine (5.6 ml; 0.04 mole) was added and it was stirred at 0°C for half an hour. Then ethyl chloroformiate (4.2 ml; 0.04 mole) was added and it was stirred in ice for further half an hour. Then 67 ml of benzylamine solution (9.6 ml; 0.088 mole benzylamine in 100 ml methylene chloride) were added dropwise to pH 7.5 and it was stirred at room temperature for further two hours. The precipitate was sucked off and to the liquor, which was rinsed with 1N HCl, with water and dried (Na₂SO₄), glacial acetic acid (91.2 ml) and hydrogen peroxide (50 ml) were added and it was stirred at room temperature. When the reaction was completed, the reaction mixture was rinsed with water, sodium hydrogen carbonate solution, salty water, and it was dried and evaporated to dryness. By recrystallization of the evaporated residue from methanol, a product (8.2 g) was obtained.

The evaporated methanolic liquor after crystalization (4.5 g) was purified by flash chromatography with methylene chloride and methylene chloride-acetone (10:0.3). There were obtained additional 1.2 g of the product. Totally, there were obtained 9.3 g (50.2%).
M.p. 158-160 °C (MeOH).
   Rf 0.2 methylene chloride-acetone (10:0.3)

Spectroscopic data were identical to those in Example 2.

### Example 4

### 3-Bromo- 7,7-dibromo-3-methyl-cepham-4-carboxylic acid benzyl ester

Benzyl ester of 6,6-dibromopenicillanic acid sulfoxide (13.95 g; 0.03 mole) in acetonitrile (100 ml) was stirred under reflux condenser at boiling temperature. When the reaction was completed (10 hours), which was established by thin layer chromatography on a silica gel layer (methylene chloride), the reaction mixture was evaporated to a dry residue and purified by flash chromatography in a sequence of solvent systems: a) petroleum ether-methylene chloride (8:2) b) petroleum ether-methylene chloride (6:4), c) methylene chloride, and then a product was isolated.
M.p. 93-95 °C
   Rf 0.60 (methylene chloride)
IR(KBr) νₘₐₓ: 1787 (vs), 1740 (s), 1333 (m), 1296 (m), 1228 (m), 1188(m), 1122 (m) cm⁻¹.
¹H NMR (CDCl₃, 300 MHz) δ: 1.79 (3H, s, Me), 2.79 and 3.53 (2H, ABq, J=15 Hz, H-C₂-H), 4.87 (1H, s, C₄-H), 5.15 and 5.28 (each 1H d , J=12 Hz, CH₂Ph), 5.55 (1H, s, C₆-H), 7.32-7.33 (5H, 2s, Ar) ppm.
¹³C NMR (CDCl₃, 300 MHz) APT δ: 29.80 (Me), 38.58 (C-4), 54.63 (C-3), 56.68 (C-7), 62.41 (C-2), 65.92 (C-6), 68.24 (CH₂Ph), 128.78-129.23 (Ph), 133.90 (C-Ph), 158.63 (C-8) and 165.84 (COO).
m/e M⁺ 525 (3 Br), 312 (2 Br), 91.

### Example 5

### 7,7-Dibromo-3-methyl ceph-3-em

Benzyl ester of 6,6-dibromopenicillanic acid sulfoxide (13.95 g; 0.03 mole) was dissolved in acetonitrile (150 ml) and tetrabutylammonium bromide (19.32 g; 0.06 mole) was added. The reaction mixture was stirred under reflux condenser at boiling temperature and when the starting substance disappeared from a thin layer of silica gel (10 hours), the reaction mixture was evaporated to dryness. To the dry residue methylene chloride and water were added, the layers were well shaken and separated. The methylene chloride layer was rinsed with water, sodium hydrogen carbonate solution and water, dried and evaporated to dryness. The reaction mixture was purified by flash chromatography with a solvent mixture petroleum ether-methylene chloride (7:3), whereby a product was obtained.
M.p. 101-103 °C (ether).
   Rf 0.55 methylene chloride
IR(KBr) νₘₐₓ: 1794 (vs), 1771 (vs), 1747 (s), 1390 (s), 1375(s) 1261 (w); 1107 (w), 609 (s) cm⁻¹.
¹H NMR (CDCl₃, 300 MHz) δ: 1.83 (3H, s, Me), 3.11 and 3.51 (2H, ABq, J=18 Hz, C₂-H), 5.16 (1H, s, C₄-H), 6.53 (1H, s, C₆-H) ppm.
¹³C NMR (CDCl₃, 300 MHz) APT δ: 20.95 (Me), 29.96 (C-2), 53.51 (C-7), 67.34 (C-6), 116.34 (C-4), 119.93 (C-3), 200.60 (C-8).

### Example 6

### 7,7-Dibromo-3-methyl ceph-3-em

Benzyl ester of 6,6-dibromopenicillanic acid sulfoxide (13.95 g, 0.03 mole) was dissolved in toluene (150 ml) and tetrabutylammonium bromide (19.32 g, 0.06 mole) was added. The reaction mixture was stirred under reflux condenser at boiling temperature until the starting substance dissapeared (2 hours), which was established by thin layer chromatography on a silica gel layer (methylene chloride), and then the reaction mixture was evaporated to dryness. To the dry residue methylene chloride and water were added, the layers were well shaken and separated. The methylene chloride layer was rinsed with water, a sodium hydrogen carbonate solution and water, then it was dried and evaporated to dryness. The reaction mixture was purified by flash chromatography with a mixture of solvents petroleum ether-methylene chloride (7:3), whereby a product was obtained.
M.p. 101-103°C (ether).
   Rf 0.55 methylene chloride

Spectroscopic data were identical to those in Example 5.

### Example 7

### 7,7-Dibromo-3-methyl ceph-3-em-1-oxide

7,7-Dibromo-3-methyl ceph-3-em (0.460 g; 1.48 mmole) was dissolved in methylene chloride (15 ml), glacial acetic acid (6.8 ml) was added, to the reaction mixture 30% hydrogen peroxide (3.5 ml) was added and it was stirred at room temperature until on thin layer of silica gel in a solvent system methylene chloride-acetone (9:1) the starting substance dissapeared (6 hours). When the reaction was completed, the reaction solution was rinsed with water, sodium hydrogen carbonate solution, and water and it was dried and evaporated to dryness. The obtained product, a mixture of isomers, was purified by chromatography on a silica gel column with the solvent system methylene chloride-acetone (9:1), whereby in the first place the first isomer with respect to sulphur was isolated.
M.p. 153-155°C
   Rf 0.40 methylene chloride - acetone (9:1)
IR (KBr) νₘₐₓ: 3422 (m), 1788 (vs), 1382 (m), 1371 (s), 1264 (s), 1064 (vs), 737 (vs) cm⁻¹.
¹H NMR (CDCl₃, 300 MHz) δ: 1.86 (3H, s, Me), 3.56 and 3.91 (2H, ABq, J=16 Hz, C₂-H), 4.89 (1H, s, C₄-H), 6.52 (1H, s, C₆-H) ppm.
¹³C NMR (CDCl₃, 300 MHz) APT δ: 20.11 (Me), 50.53 (C-7) 53.80 (C-2), 81.62 (C-4), 117.07 (C-3), 117.27 (C-6), 154.70 (C=O) ppm.

Then the second isomer with respect to sulphur was isolated.
M.p. 188-190°C
   Rf 0.30 methylene chloride-acetone (9:1)
IR (KBr) νₘₐₓ: 3422 (vs), 1773 (s), 1644 (vs), 1382 (m), 1371 (s), 1033 (vs) cm⁻¹.
¹H NMR (CDCl₃, 300 MHz) δ: 1.88 (3H, s, Me), 3.27 and 3.60 (2H, ABq, J=17.5 Hz, C₂-H), 4.64 (1H, s, C₄-H), 6.52 (1H, s, C₆-H) ppm.

### Example 8

### 7,7-Dibromo-3-methyl ceph-3-em-4-carboxylic acid benzyl ester

Benzyl ester of 6,6-dibromopenicillanic acid sulfoxide (13.95 g; 0.03 mole) was dissolved in acetonitrile (150 ml) and tetrabutylammonium bromide (19.32 g, 0.06 mole) was added. The mixture was stirred under reflux condenser at boiling temperature and when the starting substance dissapeared (10 hours), which was established by thin layer chromatography on a silica gel layer (methylene chloride), the reaction mixture was evaporated to dryness. To the dry residue methylene chloride and water were added, the layers were well shaken and separated. The methylene chloride layer was rinsed with water, a sodium hydrogen carbonate solution and water, dried and evaporated to dryness. The reaction mixture was purified by flash chromatography with a mixture of solvents 1) petroleum ether-methylene chloride (1:1) and 2) methylene chloride, and a product was isolated.
M.p. 111-113°C (ether)
   Rf 0.50 (methylene chloride)
IR (KBr)νₘₐₓ: 3070-2920 (vs), 1790 (vs), 1740 (vs), 1375 (m), 1365 (m), 1215 (m), 740 (s) cm⁻¹.
¹H NMR (CDCl₃, 300 MHz) δ: 2.24 (3H, s, Me), 3.20 and 3.29 (each 1H, d, J=16.6 Hz, C₂-H), 5.25 (1H, s, C₆-H), 5.27 and 5.31 (each 1H, d, J=12Hz, CH₂Ph), 7.30-7.43 (5H, m, Ar) ppm.
¹³C NMR (CDCl₃, 300 MHz) APT δ: 19.77 (Me), 31.62 (C-2), 56.42 (C-7), 67.33 (CH₂Ph), 70.52 (C-6), 123.06; 128.23-128.55 (Ph), 135.10 (C-Ph), 141.04; 157.72 (C=O), 161.55 (COO).
m/e M⁺ 445 (⁷⁹Br₂), 356 (⁷⁹Br₂), 270 (⁷⁹Br₂).

### Example 9

### 7,7-Dibromo-3-β-hydroxy-3-methyl cepham-4-carboxylic acid benzyl ester

Benzyl ester of 6,6-dibromopenicillanic acid sulfoxide (13.95 g; 0.03 mole) was dissolved in acetonitrile (150 ml) and the reaction mixture was stirred under reflux condenser at boiling temperature until the starting substance disappeared (10 hours), which was established by thin layer chromatography on a silica gel layer (methylene chloride), and then the reaction mixture was evaporated to dryness. To the dry residue methylene chloride and water were added, the layers were well shaken and separated. The methylene chloride layer was rinsed with water, a sodium hydrogen carbonate and water, dried and evaporated to dryness. The reaction mixture was purified by flash chromatography in a sequence of solvent systems 1) petroleum ether-methylene chloride (8:2), 2) petroleum ether-methylene chloride (6:4), and 3) methylene chloride, when a product in a 50% yield was isolated.
M.p. 138-139°C (i-PrOH)
   Rf 0.40 (CH₂Cl₂)
IR (KBr) νₘₐₓ: 3446 (m), 1784 (vs) 1736 (vs), 1394 (m), 1375 (m), 1333 (s), 1282 (m), 1149 (m), 1138 (m), 945 (m) cm⁻¹.
¹H NMR (CDCl₃, 300 MHz) δ: 1.23 (3H, s, Me), 2.57 and 3.50 (2H, ABq, J=14Hz, CH₂), 3.76 (1H, s, OH, disappears with D₂O), 4.46 (1H, s, C₄-H), 5.13 and 5.25 (each 1H, d, J=12Hz, CH₂Ph), 7.37 (5H, s, Ar) ppm.
¹³C NMR (CDCI₃, 300 MHz) APT δ: 24.14 (Me), 36.53 (C-2), 53.82 (C-7), 61.38 (C-4), 62.89 (C-3), 66.33 (C-6), 67.82 (CH₂Ph), 128.57-128.94 (Ph), 134.12 (C-Ph), 158.74 (C-8), 167.4 (COO).
m/e M⁺ 463 (2Br), 384 (Br), 91(CH₂Ph).

### Example 10

### 3-β-Bromo-7,7-dibromo-3-α-methyl cepham-4-carboxylic acid benzylamide

To benzylamide of 6,6-dibromopenicillanic acid sulfoxide (4.60 g; 0.01 mole) dissolved in acetonitrile (100 ml), tetrabutylammonium bromide (6.4 g; 0.04 mole) was added and the reaction mixture was refluxed for 12 hours. When the reaction was completed, which was established by thin layer chromatography on a silica gel layer (methylene chloride), the reaction mixture was evaporated to a dry residue and passed through a silica gel column with a solvent mixture methylene chloride-acetone (10:0.3), whereby a product was isolated.
M.p. 58-60°C (n-hexane)
   Rf 0.7 methylene chloride-acetone (10:0.3)
IR (KBr) νₘₐₓ: 3336 (m), 1785 (vs), 1677 (s), 1655 (s), 1540 (m), 1452 (m), 1399 (m), 1340 (m), 1222 (m), 1050 (m), 648 (m) cm⁻¹.
¹H NMR (CDCl₃, 300 MHz) δ: 1.89 (3H, s, Me), 2.77 and 4.06 (2H, ABq, J=14.5 Hz, H-C₂-H), 4.28 and 4.56 (2H, each 1H ABX system, J _{gem.} 14.6 Hz, J _{vic} 5.0 and 6.2 Hz, NCH₂Ph), 4.83 (1H, s, C₄-H), 5.61 (1H, s, C₆-H), 7.30-7.36 (5H, m, Ar), 7.43 (1H, m, NH) ppm.
¹³C NMR (CDCl₃, 300 MHz) APT δ: 29.69 (Me), 38.91 (C-2), 43.64 (N-CH₂Ph), 54.34 (C-7), 59.23 (C-3), 62.66 (C-4), 66.15 (C-6), 127.77-128.84 (Ph), 159.52 (C=O), 164.92 (CO-N).
m/e M⁺ 524 (⁷⁹Br₃), 445 (⁷⁹Br₂).

### Example 11

### 3-β-Bromo-7,7-dibromo-3-α-methyl cepham-4-carboxylic acid benzylamide

To benzylamide of 6,6-dibromopenicillanic acid sulfoxide (4.60 g; 0.01 mole) dissolved in toluene (100 ml), tetrabutylammonium bromide (6.4 g; 0.04 mole) was added and the reaction mixture was stirred under reflux condenser at boiling temperature. When the reaction was completed (2 hours), which was established by thin layer chromatography on a silica gel layer (methylene chloride), the reaction mixture was evaporated to a dry residue and passed through a silica gel column with a solvent mixture methlyene chloride-acetone (10:0.3), whereby a product was isolated.
M. p. 58-60°C (n-hexane)
   Rf 0.7 methylene chloride-acetone (10:0.3)

Spectroscopic data were identical to those in Example 9.

### Example 12

### 7,7-Dibromo-3-methyl-ceph 3-em-4-carboxylic acid benzylamide

Benzylamide of 6,6-dibromopenicillanic acid sulfoxide (4.60 g; 0.01 mole) was stirred in acetonitrile (100 ml) under reflux condenser at boiling temperature. When the reaction was completed, which was established by thin layer chromatography on a silica gel layer (methylene chloride), the reaction mixture was evaporated to a dry residue and passed through a silica gel column with a solvent mixture methylene chloride-acetone (10:3), whereby a product was isolated.
M.p. 57-59°C (n-hexane).
   Rf 0.60 methylene chloride-acetone (10:0.3)
IR(KBr) νₘₐₓ: 3330 (m), 1785 (vs), 1660 (s), 1535 (m), 700 (m) cm⁻¹.
¹H NMR (DMSO-d₆, 300 MHz) δ: 1.88 (3H, s, Me), 3.35 and 3.57 (2H, ABq, J=17 Hz, H-C₂-H), 4.32 and 4.48 (2H, each 1H ABX system, J _{gem.} 13.7 Hz, J _{vic} 4.9 and 5.9 Hz, NCH₂Ph), 5.56 (1H, s, C₆-H) 7.20-7.40 (5H, m, Ar), 9.07 (1H, m, NH) ppm.
¹H NMR (DMSO-d₆+D₂O, 300 MHz) δ: 1.85 (3H, s, Me), 3.32 and 3.55 (2H, each 1H d, J=17.4 Hz, H-C₂-H), 4.32 and 4.43 (2H, each 1H d, J=15 Hz, NCH₂Ph), 5.52 (1H, s, C₆-H) 7.20-7.33 (5H, m, Ar) ppm.
¹³C NMR (DMSO-d₆, 300 MHz) APT δ: 18.80 (Me), 29.88 (C-2), 57.01, 67.64, 116.62, 127.14-128.71 (Ph), 139.25 (C-Ph), 156.48, 161.50.
m/e M⁺ 444 (2Br), 365 (Br), 106, 91.

### Example 13

### 3-(β-Bromo-7,7-dibromo-3-α-methyl cepham-1,1-dioxide-4-carboxylic acid benzylamide

A mixture of 6,6-dibromopenicillanic acid sulfoxide (4.6 g; 0.01 mole) and tetrabutylammonium bromide (6.4 g; 0.02 mole) was stirred in acetonitriIe (100 ml) under reflux condenser at boiling temperature until the starting substance disappeared. When the reaction was completed (12 hours), which was established by thin layer chromatography on a silica gel layer in a solvent system methylene chloride-acetone (10:0.3), the reaction mixture was evaporated to dryness. The evaporated residue was subjected to oxidation in formic acid (28 ml) with hydrogen peroxide (11.2 ml) in such a way that the reaction mixture was refluxed for half an hour. To the mixture methylene chloride was added, the layers were separated and the methylene chloride layer was rinsed with water, sodium hydrogen carbonate, water and dried and evaporated to dryness. The evaporated residue was purified by chromatography on a column with system methylene chloride-acetone (9:1), whereby a product (1.75 g) (pale yellow foam, 31%) was isolated.
M.p.163-165°C
   Rf 0.45 methylene chloride-acetone (9:1)
IR(KBr) νₘₐₓ: 3260-90 (vs), 1797 (vs), 1670 (vs), 1560 (s), 1425 (m), 1394 (m), 1332 (m), 1051 (vs), 752 (s), 702 (s) cm⁻¹.
¹H NMR (CDCl₃, 300 MHz) δ: 1.90 (s, 3H, Me), 3.69 and 4.25 (2H, ABq, J=13 Hz, H-C₂-H) 4.37 and 4.56 (2H, each 1H ABX system, J _{gem.} 14.6 Hz, J _{vic} 5.0 and 6.0 Hz, NCH₂Ph), 4.56 (1H, s, C₄-H), 5.35 (1H, s, C₆-H), 7.14 (m, 1H, NH), 7.28-7.38 (5H, m, Ar) ppm.
¹³C NMR (CDCl₃, 300 MHz) APT δ: 30.1 (Me), 43.90 (C-2), 50.74 (C-3), 59.17 (CH₂N), 59.48 (C-7), 61.62 (C-6), 84.16 (C-4), 127.91-129.03 (Ph), 136.41 (C-Ph), 158.48 (C=O) and 164.21 (CONH).
m/e M⁺ 540 (3Br), 461 (2Br), 91.

## Claims

1. 7-Bromo- and 7,7-dibromo-cepham derivatives of general formula **I** 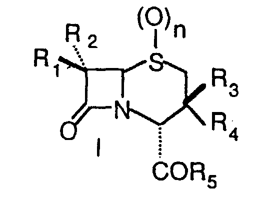 wherein the radicals have the meanings
R₁ is hydrogen or bromine,
R₂ is hydrogen or bromine,
R₃ is bromine,
R₄ is methyl, hydroxy,
R₅ is -OR₆ or -NR₇R₈, wherein R₆, R₇ and R₈ are hydrogen, alkyl, alkylaryl,
n = 0, 1, 2.

2. 7-Bromo- and 7,7-dibromo-cephem derivatives of general formula **II** 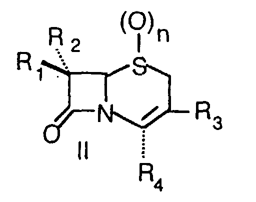 wherein the radicals have the meanings
R₁ is hydrogen or bromine,
R₂ is hydrogen or bromine,
R₃ is methyl,
R₄ is hydrogen, -COOR₅ or CONR₆R_{7,}
wherein R₅, R₆ and R₇ are hydrogen, alkyl, alkylaryl,
n = 0, 1, 2.

3. A compound according to claim 1, characterized in that
R₁ is bromine, R₂ is bromine, R₃ is bromine, R₄ is methyl, R₅ is -OR₆, wherein R₆ is benzyl, n = 0.

4. A compound according to claim 1, characterized in that
R₁ is bromine, R₂ is bromine, R₃ is hydroxy, R₄ is methyl, R₅ is -OR₆, wherein R₆ is benzyl, n = 0.

5. A compound according to claim 1, characterized in that
R₁ is bromine, R₂ is bromine, R₃ is bromine, R₄ is methyl, R₅ is -NHCH₂Ph, n = 0.

6. A compound according to claim 1, characterized in that
R₁ is bromine, R₂ is bromine, R₃ is bromine, R₄ is methyl, R₅ is -NHCH₂Ph, n = 2.

7. A compound according to claim 2, characterized in that
R₁ is bromine, R₂ is bromine, R₃ is methyl, R₄ is hydrogen, n = 0.

8. A compound according to claim 2, characterized in that
R₁ is bromine, R₂ is bromine, R₃ is methyl, R₄ is hydrogen, n = 1.

9. A compound according to claim 2, characterized in that
R₁ is bromine, R₂ is bromine, R₃ is methyl, R₄ is COOR₅, R₅ is benzyl, n = 0.

10. A compound according to claim 2, characterized in that
R₁ is bromine, R₂ is bromine, R₃ is methyl, R₄ is NHCOCH₂Ph, n = 0.

11. Process for the preparation of novel 7-bromo- and 7,7-dibromo-cepham derivatives of the general formula **I** 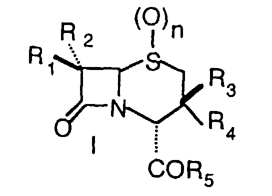 wherein the radicals have the meanings
R₁ is hydrogen or bromine,
R₂ is hydrogen or bromine,
R₃ is bromine,
R₄ is methyl, hydroxy,
R₅ is -OR₆ or -NR₇R₈, wherein R₆, R₇ and R₈ are hydrogen, alkyl, alkylaryl,
n = 0, 1, 2,
as well as
7-bromo- and 7,7-dibromo cephem derivatives of the general formula **II** 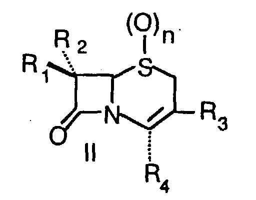 wherein the radicals have the meanings
R₁ is hydrogen or bromine,
R₂ is hydrogen or bromine,
R₃ is methyl,
R₄ is hydrogen, -COOR₅ or CONR₆R₇,
wherein R₅, R₆ and R₇ are hydrogen, alkyl, alkylaryl,
n = 0, 1, 2,
characterized in that starting from esters or amides of 6-bromo- and 6,6-dibromo-penicillanic acid sulfoxides of the general formula **III** 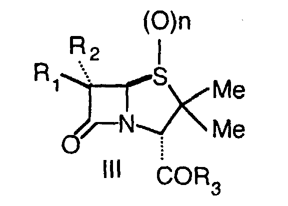 wherein the radicals have the meanings
R₁ is hydrogen or bromine,
R₂ is hydrogen or bromine,
R₃ is OR₄ or NR₅R₆, wherein R₄, R₅ and R₆ are hydrogen, alkyl, alkylaryl,
n = 1,
by heating at boiling temperature in some organic solvent e.g. acetonitrile, toluene, with or without addition of tetrabutylammonium bromide, whereupon the reaction mixture is evaporated to a dry residue which is, after treatment, purified by flash chromatography or chromatography on a silica gel column or is crystalized from a suitable solvent or from a suitable mixture of solvents, a product is isolated or the obtained product is immediately oxidized by usual agents e.g. by hydrogen peroxide with or without an addition of glacial acetic acid or formic acid or by potassium permanganate with or without an addition of glacial acetic acid.

12. Pharmaceutical or veterinary mixture active in antibacterial therapy containing as an active substance a compound according to claims 1 to 10, a suitable carrier and adjuvants.

13. A compound according to claims 1 to 10 for use in therapy of inflammatory or degenerative diseases caused by proteolytic enzymes in mammals including humans.
